# EUROPÄISCHE PATENTSCHRIFT

(11) **EP 0 991 440 B1**
(45) Veröffentlichungstag und Bekanntmachung des Hinweises auf die Patenterteilung: **29.06.2005**
(21) Anmeldenummer: 98913501.7
(22) Anmeldetag: 22.04.1998
(51) Int. Cl.: A61M 5/145, A61M 5/24

(54) **ANTRIEBSVORRICHTUNG FÜR EINEN KOLBEN IN EINEM EIN MEDIKAMENTFLUID ENTHALTENDEN BEHÄLTNIS**
PROPELLING DEVICE FOR A PISTON IN A CONTAINER CONTAINING A LIQUID MEDICAMENT
DISPOSITIF D'ENTRAINEMENT POUR UN PISTON SITUE DANS UN CONTENANT RENFERMANT UN MEDICAMENT SOUS FORME FLUIDE

(30) Priorität: 23.04.1997 DE 19717107
(43) Veröffentlichungstag der Anmeldung: 12.04.2000
(73) Patentinhaber: Disetronic Licensing AG, 3401 Burgdorf (CH)
(72) Erfinder: PETER, Daniel, CH-3172 Niederwangen (CH); KINDLER, Beat, CH-3415 Hasle-Rüegsau (CH)
(74) Vertreter: Wess, Wolfgang, Dipl.-Ing.
(86) Internationale Anmeldenummer: PCT/CH1998/000157
(87) Internationale Veröffentlichungsnummer: WO 1998/047552

(56) Entgegenhaltungen:
- EP-A- 0 293 958
- EP-A- 0 327 910
- WO-A-97/00091

## Beschreibung

Die Erfindung betrifft eine Vorrichtung zur Verabreichung eines Medikamentfluids mit einer Antriebsvorrichtung für einen Kolben in einem das Medikamentfluid enthaltenden Behältnis.

Für die Verabreichung von Medikamenten in fluider Form, insbesondere flüssiger Form, beispielsweise Insulin, kommen tragbare Injektions- und/oder Infusionsgeräte zum Einsatz. Das Medikamentfluid wird fein dosiert aus einem Fluidbehältnis mittels eines Kolbens verdrängt und verabreicht. Breiten Einsatz finden solche Geräte als Pumpgeräte und manuell zu betätigende Pens in der Insulinbehandlung. Ein Injektionspen ist beispielsweise aus der WO 93/16740 bekannt. Ein Beispiel für ein tragbares Infusionsgerät ist die Insulinpumpe H-TRON® plus der Disetronic Medical Systems AG. Der Verwender trägt das Gerät im Allgemeinen ständig bei sich, beispielsweise am Arbeitsplatz oder auch im Urlaub. Um größtmögliche Unabhängigkeit von externer Versorgung einerseits und Bewegungsfreiheit andererseits zu haben, sollte das Gerät zwar möglichst viel Medikamentfluid fassen können, aber dennoch klein sein. Die Forderung nach platzsparender Bauweise besteht im medizinischen Bereich auch grundsätzlich; so auch bei stationären Geräten und Anlagen. Ein tragbares Infusionsgerät ist ferner aus der EP 0 143 895 A1 bekannt.

Die Erfindung hat es sich zur Aufgabe gemacht, bei einer Vorrichtung zur Verabreichung eines Medikamentfluids eine Antriebsvorrichtung für einen Kolben in einem das Medikamentfluid enthaltenden Behältnis zu schaffen, die wenig Raum beansprucht und daher insbesondere als Antriebsvorrichtung für ein tragbares Medikamentverabreichungsgerät geeignet ist.

Diese Aufgabe wird durch den Gegenstand von Anspruch 1 gelöst.

Wie bekannte Antriebsvorrichtungen auch, beispielsweise die der H-TRON® plus Pumpe der Disetronic Medical Systems AG oder die des aus der WO 93/16740 bekannten Injektionspens, weist auch die erfindungsgemäße Antriebsvorrichtung eine in oder an einem Basisteil verschiebbar gelagerte Verschiebestufe auf, die bei ihrem Vorschieben einen Kolben in einem ein Medikamentfluid enthaltenden Behältnis vorschiebt und dadurch Medikamentfluid aus dem Behältnis verdrängt. Indem an der Verschiebestufe die Weglänge des Kolbenvorschubs eingestellt wird, erfolgt die Dosierung der verdrängten Fluidmenge.

Zusätzlich zur ersten Verschiebestufe ist wenigstens eine zweite Verschiebestufe vorgesehen, die dem Basisteil gegenüber und auch gegenüber der ersten Verschiebstufe in Vorschubrichtung des Kolbens verschiebbar ist, entweder manuell oder motorisch, und bei ihrem Verschieben in Vorschubrichtung des Kolbens die erste Verschiebestufe mitnimmt. Die zweite Verschiebestufe ist eine Gewindehülse. Durch die Mehrstufigkeit der Antriebsvorrichtung wird die maximale Weglänge, um die der Kolben verschoben werden kann, in mehrere Wegstücke zerlegt, nämlich ein Wegstück pro Verschiebestufe. Die wenigstens zwei Verschiebestufen sind in ihren Ausgangsstellungen zumindest einander teilweise überlappend angeordnet. Durch die Aufspaltung der maximalen Verschiebeweglänge in mehrere Wegstücke durch Kaskadierung der Antriebsvorrichtung wird die in Vorschubrichtung des Kolbens gemessene Gesamtlänge von Fluidbehältnis und Antriebsvorrichtung verringert. Die Verschiebestufen bilden vorzugsweise einen Teleskopantrieb.

Aus der WO 94/15660 und der WO 97/00091 sind Teleskopantriebe bekannt, die in ein rückwärtig offenes Medikamentbehältnis eingesetzt und am Behältnis befestigt werden. Dabei wird eine Antriebspindel motorisch drehangetrieben. Auf der Antriebspindel laufen jeweils zwei koaxial zur Antriebsspindel angeordnete, die Antriebsspindel umhüllende Verschiebestufen. Die äussere dieser beiden Verschiebestufen ist verdrehgesichert; bei der WO 97/00091 mit Hilfe einer mit der äusseren Verschiebestufe ausfahrenden Verdrehsicherung. Durch das Drehen der Antriebsspindel werden die darauf laufende mittlere Verschiebestufe und die auf der mittleren Verschiebestufe laufende, verdrehgesicherte äussere Verschiebestufe im Behältnis auf einen Behältnisauslass zu vorgeschoben. Der Kolben zum Verdrängen des Medikamentfiuids ist am vorderen Ende der äusseren Verschiebestufe befestigt.

Nach der Erfindung besteht zwischen dem Behältnis einschliesslich Kolben und der Antriebsvorrichtung keine feste Verbindung. Die Antriebsvorrichtung und das Behältnis einschliesslich Kolben sind vielmehr jeweils für sich in einem gemeinsamen Gehäuse aufgenommen, so dass entweder das Behältnis mit dem darin gehaltenen Kolben oder die Antriebsvorrichtung oder beides auf einfache Weise ausgetauscht werden kann, da nicht erst eine Verbindung der Antriebsvorrichtung mit dem Kolben und/oder mit dem Behältnis gelöst werden muss. Dies erleichtert insbesondere einen Austausch des Behältnisses, beispielsweise nach Verabreichung eines gesamten Behältnisinhalts. Die Antriebsvorrichtung kann im Gehäuse verbleiben; sie wird dabei nicht beeinflusst.

Vorzugsweise ist auch die Antriebsvorrichtung austauschbar gegen eine neue im Gehäuse aufgenommen. Das Gehäuse kann unmittelbar das genannte Basisteil bilden. In einer ebenfalls bevorzugten Ausführungsform bildet das Basisteil mit den darin aufgenommenen Verschiebestufen und einem darin bevorzugt ebenfalls gelagerten Motorantrieb einen einfach austauschbaren Antriebsmodul, der im Gehäuse befestigt ist. Die erste Verschiebestufe der Antriebsvorrichtung weist mit dem Kolben lediglich eine Andruckverbindung auf, d.h. sie drückt lose gegen den Kolben bzw. stösst nur daran an, um ihn vorzuschieben. Durch die körperliche Trennung ist es auch grundsätzlich möglich, die gleiche Antriebsvorrichtung bei unterschiedlichen Behältnisformen und auch bei unterschiedlichen Kolbenformen einzusetzen.

Bei den Verschiebestufen handelt es sich vorzugsweise um in sich starre, nur entlang einer Raumachse geradverschiebbare Bauteile. Biegsame Stufen, die bis neben das Fluidbehältnis führbar wären, könnten grundsätzlich jedoch auch eingesetzt werden.

In ihrer Ausgangsstellung umgibt die Gewindehülse die erste Verschiebestufe. Diese Anordnung der Verschiebestufen hat zudem den Vorteil der geringsten Ausdehnung quer zur Vorschubrichtung. Sie findet mit Vorteil sowohl in Injektionspens als auch in Pumpengeräten Verwendung.

Falls neben dem Fluidbehältnis Raum zur Verfügung steht, so beispielsweise bei der bereits genannten Pumpe H-TRON® plus, so kann vorteilhafterweise wenigstens eine Verschiebestufe dort angeordnet werden. Während die Achse, entlang der die eine Verschiebestufe in Vorschubrichtung des Kolbens verschoben wird, in der Verlängerung der Kolbenvorschubrichtung liegt, ist die Verschiebeachse der anderen Verschiebestufe dazu parallel beabstandet.

Die Verschiebestufen werden durch Spindeltriebe gegenüber dem Basisteil und auch relativ zueinander verschoben. Der Gewindeeingriff der Spindeltriebe ist vorzugsweise so nah als möglich zum Kolben angeordnet. Indem für das Verschieben der Verschiebestufen untereinander und schliesslich gegenüber dem Basisteil jeweils ein Spindeltrieb verwendet wird, wird eine an einer Stelle manuell oder motorisch in die Antriebsvorrichtung eingeleitete Drehbewegung in eine sich fortsetzend addierende Verschiebebewegung übertragen. Durch die Verwendung von Spindeltrieben lässt sich der Verschiebeweg präzise einstellen. Zusätzlich kann ein Spindeltrieb auch die Funktion einer Lagerung zwischen den einzelnen Verschiebestufen erfüllen.

Nach einem Ausführungsbeispiel ist eine der beiden Verschiebestufen fest mit einem Drehantrieb verbunden. Die beiden hintereinander in Serie geschalteten Spindeltriebe weisen gegenläufige Gewinde auf. Die pro Umdrehung der drehangetriebenen Verschiebestufe zurückgelegte Gesamtweglänge ist dann stets gleich der Summe der Verschiebeweglängen beider derart gekoppelten Verschiebestufen. Bei gleichen Gewindesteigungen beispielsweise wird so eine Verschiebeweglänge erzielt, die der doppelten Gewindesteigung jeder einzelnen Verschiebestufe entspricht.

Nach einem anderen Ausführungsbeispiel sind die Gewinde zweier hintereinander geschalteter Spindeltriebe gleichsinnig. Die eine Verschiebestufe wird von dem sie drehantreibenden Spindelantriebsglied vorgeschoben oder bei der Drehbewegung mitgenommen. Soweit sie verschoben wird, nimmt sie dabei die nächste Verschiebestufe einfach mit. Soweit sie vom Spindelantriebsglied einfach mitgedreht wird, erzeugt ihre eigene Drehbewegung eine erzwungene Verschiebebewegung der gegen ein Mitdrehen verdrehgesicherten nachfolgenden Verschiebestufe. Diese Art der Spindeltriebkaskadierung erlaubt eine besonders präzise Einstellung der Verschiebeweglänge der Antriebsvorrichtung.

Obgleich die Erfindung in erster Linie bei tragbaren Infusions- und/oder Injektionsgeräten Verwendung findet, kann sie mit Vorteil auch bei stationären Anlagen eingesetzt werden.

Nachfolgend werden bevorzugte Ausführungsbeispiele der Erfindung anhand von Zeichnungen erläutert. Es zeigen:
- Figur 1: ein erstes Ausführungsbeispiel einer erfindungsgemässen Antriebsvorrichtung in Draufsicht,
- Figur 2: den Längsschnitt C-C nach Figur 1,
- Figur 3: den Längsschnitt D-D nach Figur 1,
- Figur 4: den Längsschnitt wie Figur 2, wobei die Antriebsvorrichtung sich jedoch in ihrer voll ausgefahrenen Stellung befindet,
- Figur 5: den Längsschnitt wie Figur 3, wobei die Antriebsvorrichtung sich jedoch in ihrer voll ausgefahrenen Stellung befindet,
- Figur 6: die Antriebsvorrichtung nach den Figuren 1-5 in einer perspektivischen Gesamtsicht,
- Figur 7: die Antriebshülse der Antriebsvorrichtung nach den Figuren 1 bis 6,
- Figur 8: die Gewindehülse der Antriebsvorrichtung nach den Figuren 1 bis 6,
- Figur 9: die Gewindestange der Antriebsvorrichtung nach den Figuren 1 bis 6,
- Figur 10: die Verdrehsicherung der Antriebsvorrichtung nach den Figuren 1 bis 6,
- Figur 11: ein zweites Ausführungsbeispiel einer erfindungsgemässen Antriebsvorrichtung im Längsschnitt,
- Figur 12: die Antriebsvorrichtung nach Figur 11 in einem anderen Längsschnitt,
- Figur 13: ein Injektionsgerät mit einer erfindungsgemässen Antriebsvorrichtung,
- Figur 14: ein Infusionsgerät mit einer erfindungsgemässen Antriebsvorrichtung,
- Figur 15: ein drittes Ausführungsbeispiel einer erfindungsgemässen Antriebsvorrichtung und
- Figur 16: die Verdrehsicherung der Antriebsvorrichtung nach Figur 15.

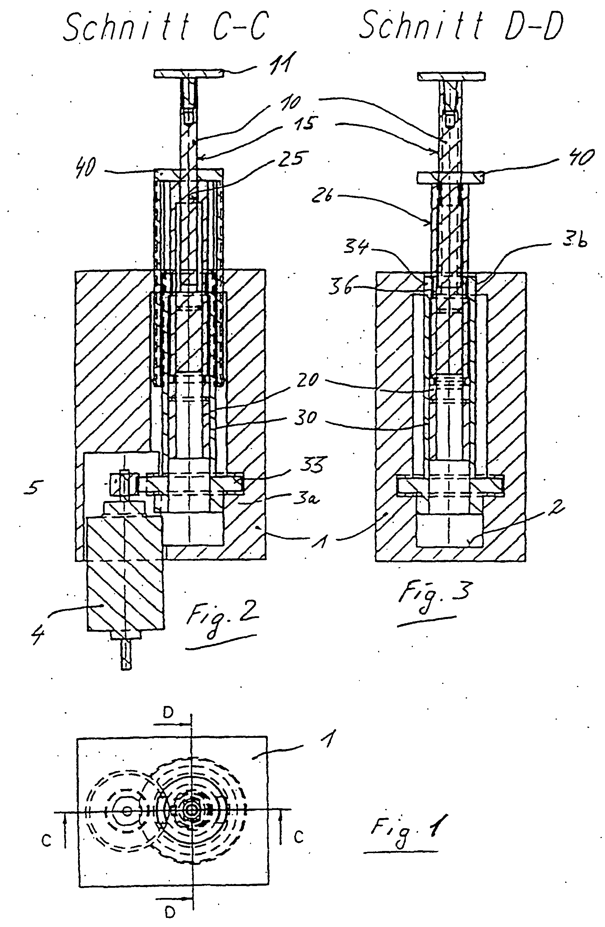

In der in Figur 1 dargestellten Draufsicht auf eine Antriebsvorrichtung sind die Lagen der in den Figuren 2 und 3 dargestellten Längsschnitte der gleichen Antriebsvorrichtung eingezeichnet.
Die Antriebsvorrichtung weist ein Basisteil 1, zwei dem Basisteil 1 gegenüber geradverschiebbare Verschiebestufen 10 und 20, ein im Basisteil 1 drehgelagertes, axial fixiertes Drehantriebsglied 30 und einen das Drehantriebsglied 30 drehantreibenden Motor 4 als ihre Hauptkomponenten auf. Die erste Verschiebestufe 10 ist als Gewindestange mit einem Aussengewinde 15 ausgebildet. Die zweite Verschiebestufe 20 ist eine Gewindehülse mit einem Innengewinde 25 und einem Aussengewinde 26. Das Drehantriebsglied 30 ist ebenfalls hohlzylindrisch und wird im folgenden als Antriebshülse bezeichnet. Sie weist ein Innengewinde 36 an einem Kopfbereich und ein Mitnehmerrad 33 an einem Fussbereich auf.

Das Mitnehmerrad 33 kämmt mit einem auf der Welle des Motors 4 sitzenden Zahnrad 5. Die Gewindestange 10 und die Gewindehülse 20 bilden über ihre Gewinde 15 und 25 einen ersten Spindeltrieb. Die Gewindehülse 20 und die Antriebshülse 30 bilden über ihre Gewinde 26 und 36 einen zweiten Spindeltrieb. Die beiden Hülsen 20 und 30 umgeben die Gewindestange 10 konzentrisch mit einer gemeinsamen Mittellängsachse, die gleichzeitig in Vorschubrichtung der Antriebsvorrichtung weist. In diese Vorschubrichtung verschiebt die Antriebsvorrichtung bei Ihrem eigenen Vorschieben einen Kolben, der in einem eine Medikamentflüssigkeit enthaltenden Reservoir bzw. Behältnis, beispielsweise eine vorkonfektionierte Ampulle, aufgenommen ist, indem die Gewindestange 10, als vorderste bzw. erste Verschiebestufe mit einem vorderen Flansch 11 gegen den Kolben drückend diesen Kolben in Richtung auf einen Auslass des Behältnisses zu vorschiebt und dadurch Flüssigkeit aus dem Behältnis verdrängt. Das Basisteil 1 fixiert dabei die Antriebsvorrichtung relativ zu dem Behältnis. Das Basisteil 1 kann an einem Gestell oder in einem Gehäuse befestigt sein oder selbst das Gestell oder das Gehäuse bilden.

Die Antriebshülse 30 ist im Basisteil 1 in einer Lagerstelle 3a, vorzugsweise ein Gleitlager, um die Mittellängsachse der Antriebsvorrichtung, die gleichzeitig deren Drehachse bildet, drehgelagert sowie axial und radial fixiert. Eine radiale Lagerstelle 3b für die Antriebshülse 30 befindet sich im oberen Teil des Basisteils 1. Die Gewindehülse 20 ist in der Antriebshülse 30 über den zwischen den Gewinden 26 und 36 gebildeten zweiten Spindeltrieb abgestützt, d.h. die Gewindehülse 20 ist über den zweiten Spindeltrieb relativ zur Antriebshülse 30 verschiebbar und im zweiten Spindeltrieb auch frei drehbar.

Die Gewindestange 10 wird gegen ein Verdrehen gegenüber dem Basisteil 1 gesichert. Die Verdrehsicherung erfolgt durch eine Verdrehsicherungsgabel 40, die gegenüber der -Gewindestange 10 längsverschiebbar, jedoch nicht verdrehbar ist, und die ihrerseits im Basisteil 1 verdrehsicher und entlang der Mittellängsachse der Antriebsvorrichtung gleitgeführt ist.

Der Vorschub der Gewindestange 10 erfolgt daher folgendermassen:

Die Drehbewegung des Motors wird über das Stimraduntersetzungsgetriebe 5, 33 auf die Antriebshülse 30 übertragen. Die Drehbewegung der Antriebshülse 30 wird über den zwischen den Gewinden 36 und 26 gebildeten zweiten Spindeltrieb auf die Gewindehülse 20 übertragen. In Abhängigkeit von den auf die Gewindehülse 20 wirkenden Reibungskräften wird die Gewindehülse 20 entweder drehend mitgenommen oder durch den Spindeltrieb mit den Gewinden 26, 36 entlang ihrer Drehachse verschoben. Die Bewegung der Gewindehülse 20 kann auch eine zusammengesetzte Verschiebe-Drehbewegung sein. Soweit die Gewindehülse 20 verschoben wird, nimmt sie die Gewindestange 10 einfach mit. Soweit die Gewindehülse 20 mitverdreht wird, erzeugt die Drehbewegung der Gewindehülse 20 über den bei den Gewinden 15 und 25 gebildeten zweiten Spindeltrieb infolge der Verdrehsicherung der Gewindestange 10 eine Vorschubbewegung der Gewindestange 10 gegenüber der Gewindehülse 20. Um diese Bewegungscharakteristik zu erhalten sind die Gewinde 26 und 15, d.h. die Gewinde, über die die beiden Verschiebestufen 10 und 20 jeweils angetrieben werden, gleichsinnig.

In den Figuren 2 und 3 ist die Antriebsvorrichtung in einer Stellung gezeigt, in der sie teilweise aus einer Ausgangsstellung im Basisteil 1 herausgefahren worden ist. In der Ausgangsstellung sind die beiden überschobenen Verschiebestufen 10 und 20 in einem Hohlraum des Basisteils 1 aufgenommen. In dieser Ausgangsstellung stossen die beiden Verschiebestufen 10 und 20 jeweils mit ihren hinteren Stirnseiten am Grund des Hohlraums an einer Abschlussfläche 2 an.

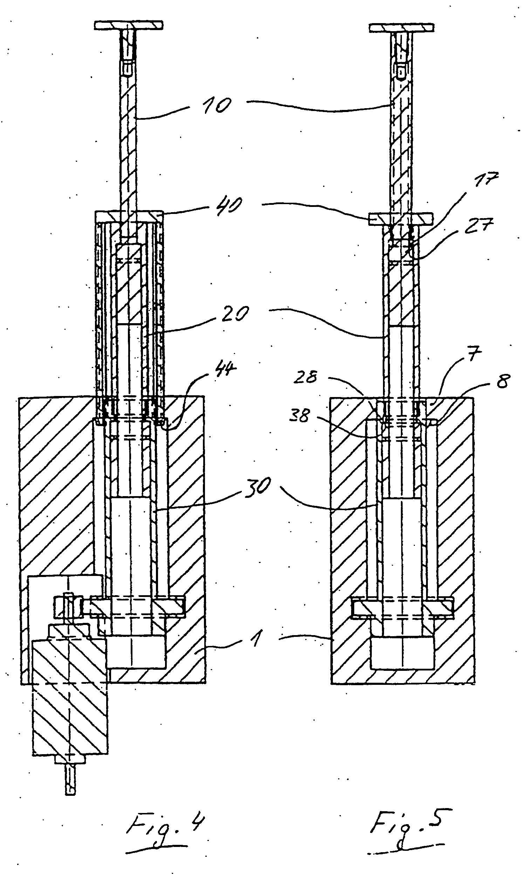

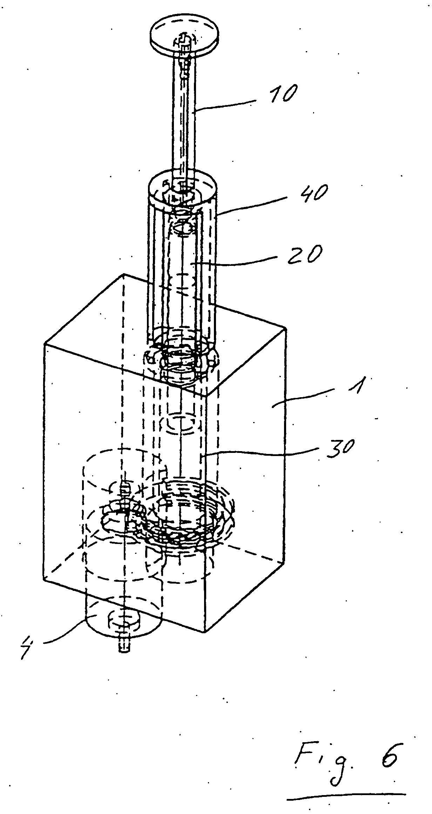

Die beiden Figuren 4 und 5 zeigen die Antriebsvorrichtung in den gleichen Schnitten wie die Figuren 2 und 3, jedoch in der voll ausgefahrenen Stellung. In dieser Stellung ist die Antriebsvorrichtung in Figur 6 auch in einer perspektivischen Gesamtsicht dargestellt.

Die vordere Endstellung der Gewindestange 10 in der Gewindehülse 20 wird durch eine Anschlagpaarung 17, 27 und die vorderste Endstellung der Gewindehülse 20 in der Antriebshülse 30 wird durch eine Anschlagpaarung 28, 38 gebildet (Figur 5). Der Anschlag 27 und der Anschlag 38 werden jeweils als von den inneren Umfangsflächen der Hülsen 20 und 30 radial nach innen abragende, umlaufende Schultern gebildet, während die entsprechenden Gegenanschläge 17 und 27 durch verdickte Ringbereiche der Gewindestange 10 bzw. der Gewindehülse 20 gebildet werden.

Ein drittes Anschlagpaar 8, 44 verhindert ein Herausfallen der Verdrehsicherungsgabel 40. Die Gabel 40 kann auch an der Hülse 20 fixiert werden. Der Anschlag 8 wird durch am vorderen Ende des Hohlraums des Basisteils 1 nach innen, auf die Verschiebeachse zu ragende Schultern 7 gebildet. Die Verdrehsicherungsgabel 40 weist entsprechende, widerhakenartig radial nach aussen abstehende Vorsprünge 44 an ihrem hinteren Ende auf.

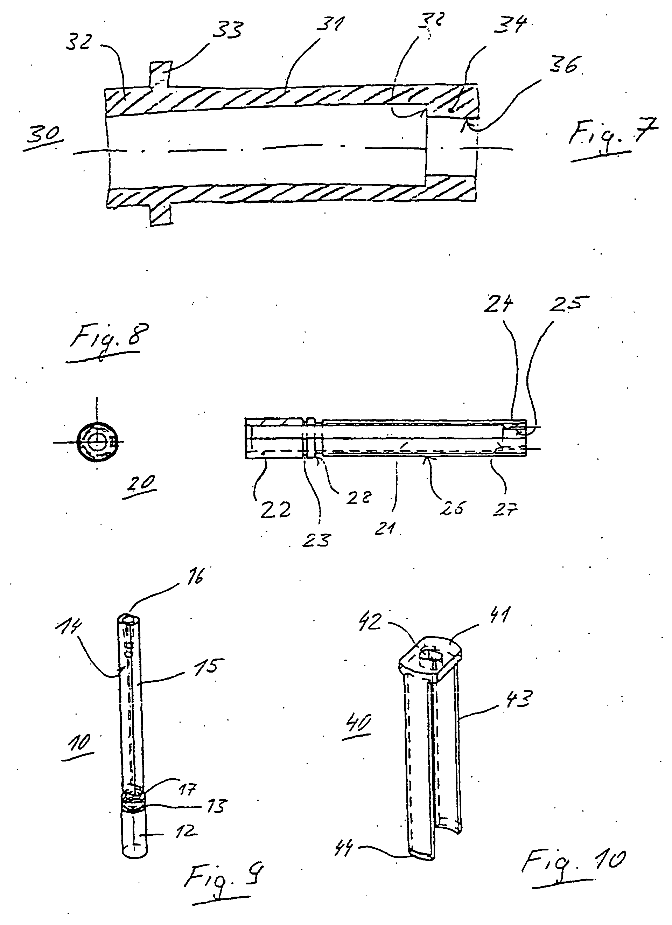

Figur 7 zeigt einen Längsschnitt der Antriebshülse 30. Diese besteht im wesentlichen aus einem einfachen, hohlzylindrischen Grundkörper 31, der in einem Fussbereich 32 der besseren Führung der Antriebshülse 30 wegen verdickt ist, so dass die äussere Mantelfläche des Fussbereichs 32 im dort kreiszylindrischen Hohlraum des Basisteils 1 beim Drehen der Antriebshülse 30 gleitet und eine zusätzliche radiale Stabilisierung zum Lager 3 darstellt. Das Mitnehmerrad 33 ist ein einfaches Stirnzahnrad, das durch eine radial um die Mantelfläche der Antriebshülse 30 umlaufende Schulter mit Zahnkranz gebildet wird. An ihrem vorderen Ende ist die Antriebshülse 30 innerhalb eines radial nach innen vorspringenden Schulterbereichs 34 mit dem Gewinde 36 versehen, das ein Feingewinde, ein mehrgängiges Gewinde mit grosser Steigung oder sogar ein Regelgewinde sein kann. Die dem Fussbereich 32 zugewandte Stirnfläche 38 ces Schulterbereichs 34 bildet den Anschlag für die Gewindehülse 20.

Die Gewindehülse 20 ist in Figur 8 dargestellt. Sie besteht ebenfalls im wesentlichen aus einem einfachen, hohlzylindrischen Grundkörper 21. Vom vordersten Ende des Grundkörpers 21 ausgehend ist die Gewindehülse 20 über den weitaus grösseren Teil ihrer Länge mit dem Gewinde 26 versehen, das im Gewinde 36 der Antriebshülse 30 läuft. Der hintere Bereich 22 der Gewindehülse 20 ist ein einfacher Kreisringzylinder, dessen Aussendurchmesser etwas grösser ist als der Aussendurchmesser des Bereichs mit dem Gewinde 26. Durch diese Verdickung wird der Gegenanschlag 28 für den Anschlag 38 der Antriebshülse 30 gebildet. Der Fussbereich 22 wird in der Antriebshülse 30 gleitgeführt. Er ist ferner umlaufend bei 23 ausgenommen. Die Ausnehmung 23 dient als Sitz für einen Dichtring. Am vorderen Ende weist die Gewindehülse 20 einen radial nach innen vorstehenden Schulterbereich 24 auf. Im Schulterbereich 24 ist das Gewinde 25 ausgebildet, für das das zum Gewinde 36 Gesagte ebenfalls gilt. Die zum Fussbereich 22 weisende Stirnfläche der Schulter 24 dient als Anschlag 27 für die Gewindestange 10.

Auch die in Figur 9 dargestellte Gewindestange 10 wird durch einen kreiszylindrischen Grundkörper gebildet. Ein einfach kreiszylindrischer Fussbereich 12 ist wiederum leicht verdickt gegenüber dem wesentlichen längeren Gewindebereich. Auch der Fussbereich 12 dient als Gleitführung beim Verschieben der Gewindestange 10 in der Gewindehülse 20. In einer ringförmig umlaufenden Ausnehmung 13 im Fussbereich 12 sitzt im eingebauten Zustand ein Dichtring. Der Bereich mit dem Gewinde 15 ist an zwei gegenüberliegenden Seiten 14 abgeflacht. An ihrem vorderen Ende ist die Gewindestange 10 mit einer stirnseitigen Sackbohrung 16 versehen, in die der Flansch 11 eingeschraubt wird. Die Abflachung 34 verhindert im Zusammenwirken mit der Verdrehsicherungsgabel 40 ein Verdrehen der Gewindestange 10 gegenüber dem Basisteil 1.

Figur 10 schliesslich zeigt die Verdrehsicherungsgabel 40. Sie ist eine an einem Ende offene und am anderen Ende mittels einer Scheibe 41 abgeschlossene Hülse, die über ihre gesamte Länge mit Schlitzen, im Ausführungsbeispiel zwei Schlitze, versehen ist. Durch die beiden Schlitze bzw. die beiden streifenförmig in Längsrichtung ausgenommenen Bereiche hat sie die Form einer zweiharkigen Gabel. Die Scheibe 41 ist mit einem Durchlass 42 versehen, der im eingebauten Zustand von der Gewindestange 10 durchstossen wird. Der Durchlass 42 wird durch zwei ebene Umfangsflächen, die durch Ringzylinderflächen verbunden sind, begrenzt. Die Gewindestange 10 wird somit im Bereich ihres Gewindes 15 und ihrer beiden flachen Führungsflächen 14 im Durchlass 42 in axialer Richtung gleitgeführt, kann sich jedoch der Verdtehsicherungsgabel 40 gegenüber nicht drehen. Die beiden axialen Fortsätze 43 werden im Basisteil 1 eng gleitgeführt, so dass die Verdrehsicherungsgabel 40 sich nicht ihrerseits dem Basisteil 1 gegenüber drehen, sondern lediglich in Längsrichtung gleiten kann. Auf diese Weise wird ein Verdrehen der Gewindestange 10 dem Basisteil 1 gegenüber verhindert. Die widerhakenförmigen Fortsätze 44 am hinteren Ende der Verdrehsicherungsgabel 40 verhindern, wie bereits erwähnt, dass die Verdrehsicherungsgabel 40 aus dem Basisteil 1 herausfallen kann.

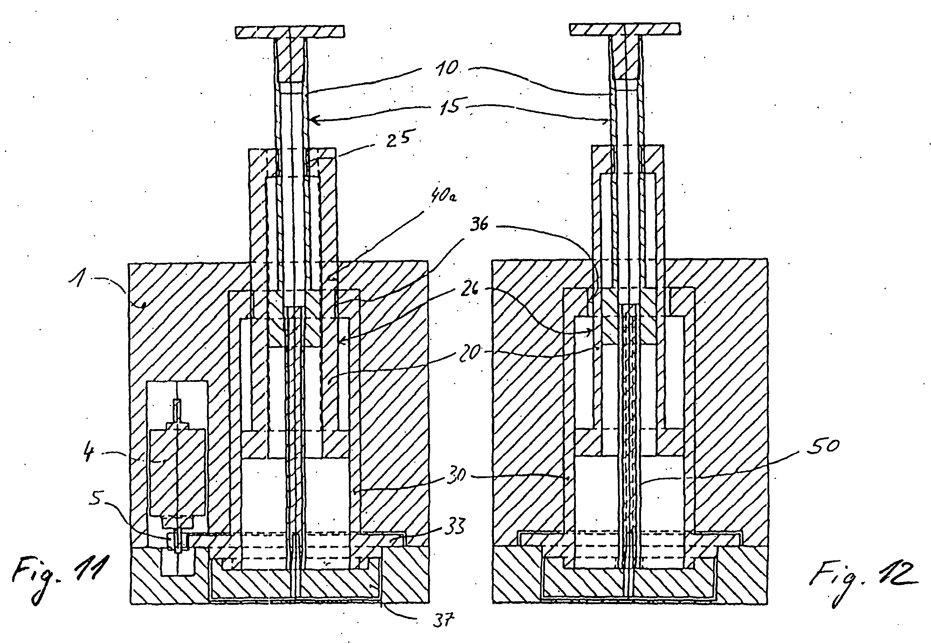

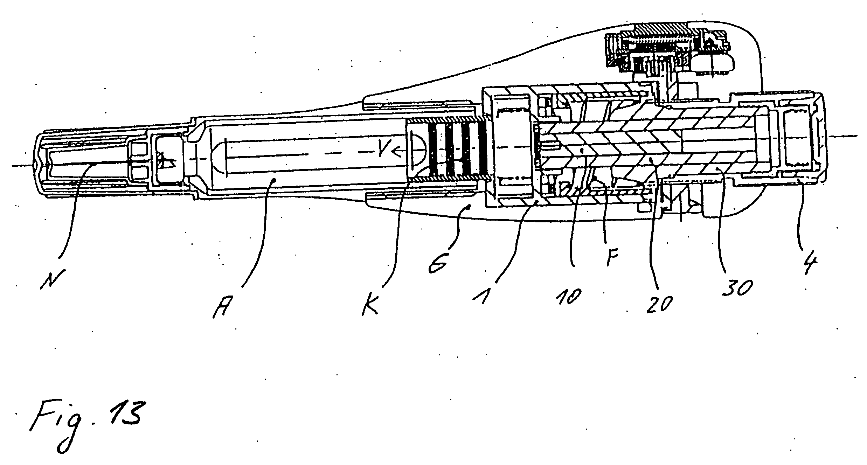

In den Figuren 11 und 12 ist eine weitere Antriebsvorrichtung dargestellt, bei der jedoch die Gewindestange 10 unmittelbar drehangetrieben wird und die Gewindehülse 20 gegen jegliches Verdrehen dem Basisteil 1 gegenüber gesichert ist. Die Antriebshülse 30 wird von dem Motor 4 drehangetrieben. Innerhalb der Antriebshülse 30 ist mit dieser drehfest verbunden ein stangenförmiges Drehantriebsmittel 50 für die erste Gewindestange 10 vorgesehen. Diese Drehantriebs- oder Mitnehmerstange 50 ragt von einem an der hinteren Stirnseite der Antriebshülse 30 befestigten Deckel in Vorschubrichtung vor und in die Gewindestange 10 hinein. Die Drehantriebsstange 50 ist ihrerseits mehrstufig, im Ausführungsbeispiel entsprechend der Anzahl der bewegbaren Verschiebestufen ist sie zweistufig in der Art eines Teleskops ausgeführt, das dem Ausfahren der Gewindestange 10 folgt. Die Gewindehülse 20 wird durch eine Verdrehsicherung 40a, die als Gleitfläche unmittelbar am Basisteil 1 vorgesehen ist, gegen ein Verdrehen relativ zum Basisteil 1 gesichert. Eine Drehung der Antriebshülse 30 wird deshalb stets in eine Verschiebebewegung der Gewindehülse 20 übertragen. Jede Drehung der Antriebshülse 30 geht wegen der drehfesten Verbindung mit einem gleichen Drehen der Drehantriebsstange 50 und damit der Gewindehülse 10 einher.

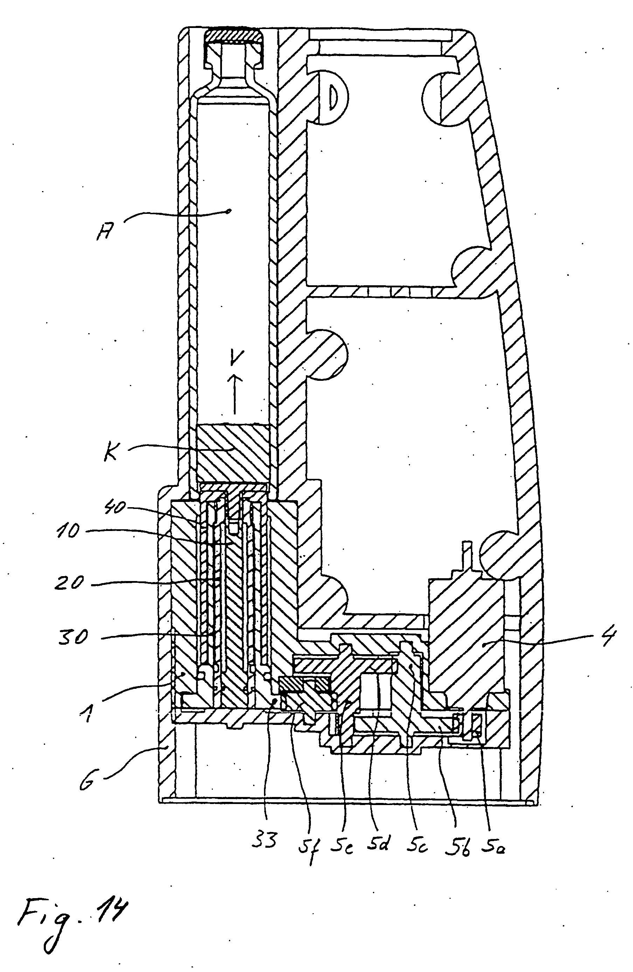

Figur 13 zeigt einen sogenannten Pen, wie er insbesondere zur Insulininjektion Verwendung findet. Im Gehäuse G des injektionspens ist eine die Medikamentflüssigkeit enthaltende Ampulle A mit einem Kolben K aufgenommen. Der Kolben K ist in eine Vorschubrichtung V auf einen Ampullenauslass zu verschiebbar und verdrängt bei seinem Vorschieben eine zuvor genau dosierte Flüssigkeitsmenge aus der Ampulle A über ein Anschlussstück in und durch eine Nadel N. Der Kolben K ist in der Ampulle A gehalten, d.h. er wird zusammen mit der Ampulle A entnommen und gegebenenfalls gegen eine neue Ampulle mit einem neuen Kolben ausgetauscht. Das Basisteil 1 dient zusammen mit einem Dosier- und Betätigungsknopf 4 zur Aufnahme der Antriebsvorrichtung und ist unabhängig vom Behältnis A im Gehäuse G aufgenommen und in seiner Lage relativ zum Behältnis A und zum Kolben K fixiert. Das Basisteil 1 dient mit einem vorderen Rand jedoch zum Fixieren der Ampulle A. Nach dem Aufschrauben des Gehäuses G werden so eine vordere und eine hintere Gehäusehülse erhalten, wobei die Ampulle A in der vorderen und die Antriebsvorrichtung in der hinteren Gehäusehälfte aufgenommen sind. Im zusammengeschraubten Zustand drückt dann das Basisteil 1 gegen den hinteren Ampullenrand, so dass die Ampulle A in Längsrichtung verschiebegesichert im Gehäuse G sitzt.

Der Vorschub des Kolbens K wird durch Vorschieben einer Gewindestange 10 bewirkt, die beim Vorschieben auf die Kolbenrückseite drückend diesen Kolben K in der Ampulle A vorschiebt. Die Gewindestange 10 bildet die erste Verschiebestufe eines Teleskopantriebs. Die zweite Verschiebestufe wird durch eine Gewindehülse 20 gebildet, in der die Gewindestange 10 mittels eines ersten Spindeltriebs läuft. Die Gewindehülse 20 wird ihrerseits von einer Antriebshülse 30 umgeben, mit der sie einen zweiten Spindeltrieb zu ihrem Verschieben in und entgegen der Vorschubrichtung V bildet. Die Antriebshülse 30 ist im Gehäuse 1 drehgelagert. Die Antriebshülse 30 wird dem Gehäuse G gegenüber manuell mittels des Dosier- und Betätigungsknopfs 4 um die in Vorschubrichtung V weisende Mittellängsachse der Antriebsvorrichtung 10, 20, 30 zum Einstellen der zu verabreichenden Insulindosis verdreht und anschliessend zusammen mit der Gewindestange 10 und der Gewindehülse 20 entlang der Längsachse vorgeschoben. Durch eine dabei zusammengedrückte Feder F wird sie nach der Injektion bzw. nach der Betätigung eines Resetknopfs wieder in ihre Ausgangsstellung für die nächste Injektion zurückgeschoben.

Die Dosierung und manuelle Betätigung des Injektionspens erfolgt wie bei bekannten Pens auch. Diesbezüglich sei daher beispielhaft auf die Beschreibung solch eines Injektionspens in der WO 93/16740 verwiesen. Im Unterschied zu den bekannten Injektionspens wird im Pen der Figur 13 jedoch eine erfindungsgemässe Antriebvorrichtung für den Kolben K verwendet. Die Antriebsvorrichtung dieses Verwendungsbeispiels entspricht dem ersten Ausführungsbeispiel.

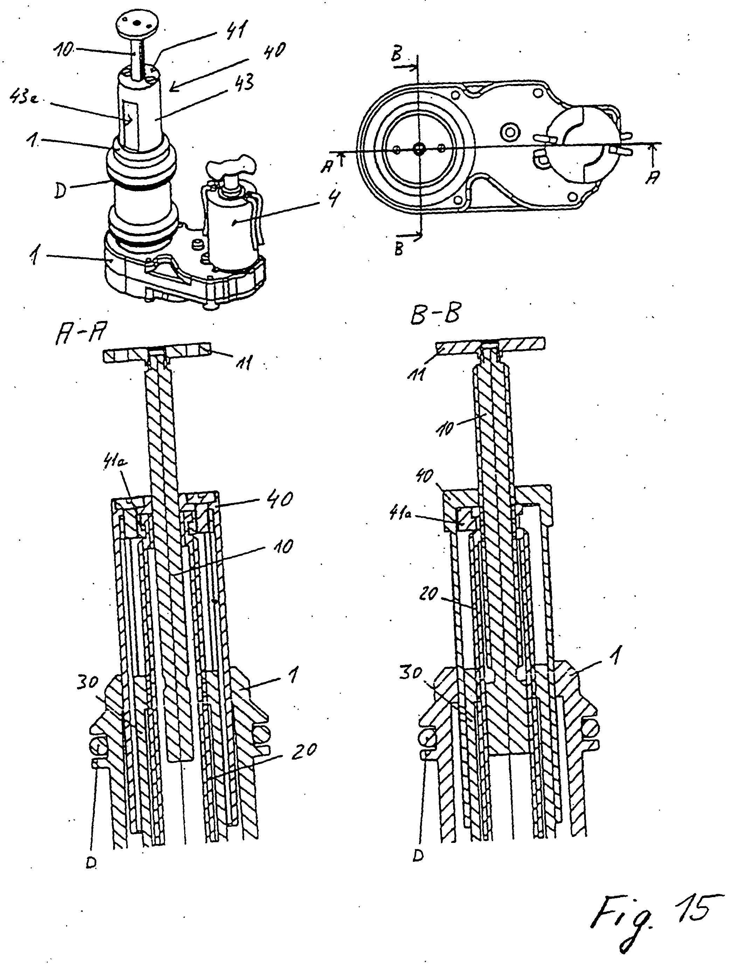

Figur 14 schliesslich zeigt ein tragbares Infusionsgerät, insbesondere für die Insulinbehandlung, mit einer motorisch angetriebenen Antriebsvorrichtung. Beispielhaft wird die Antriebsvorrichtung nach den Figuren 1 bis 10 verwendet. Das Basisteil 1 mit der Antriebshülse 30, zwei Verschiebestufen 10 und 20 einschliesslich Verdrehsicherungsgabel 40 ist im Pumpengehäuse G fixiert; nicht zuletzt durch gegenseitig angepasste Formgebung.

Die Ampulle A mit dem darin gehaltenen Kolben K ist der einfachen Austauschbarkeit wegen im Beispiel der Figur 14 in das Gehäuse G einsteckbar bzw. herausziehbar. Eine Verbindung, die erst gelöst werden müsste, besteht nicht. Die Antriebsvorrichtung kann beim Austausch der Ampulle A im Gehäuse G verbleiben bzw. unabhängig von der Ampulle A ausgetauscht werden. Das Basisteil 1 dient jedoch wie bereits im Ausführungsbeispiel der Figur 13 als Anschlagmittel, d.h. zur Fixierung der Ampulle A in Ampullenlängsrichtung. Die Ampulle A ist in einem Einsteckschacht des Gehäuses G bis gegen diesen Anschlag hin und her verschiebbar und dabei in Längsrichtung geführt aufgenommen. Nach dem Einschieben wird sie gegen ein Vorschieben im Schacht mit Hilfe eines am Gehäuse G zu befestigenden Verschlussmittels gesichert.

Beim Ausfahren aus der in Figur 14 dargestellten hintersten Stellung der Antriebsvorrichtung schiebt die Gewindestange 10 den Kolben K in der Ampulle A in Richtung V auf den Ampullenauslass zu, der im Ausführungsbeispiel jedoch noch von einer Membran dicht verschlossen ist. Angetrieben wird die Antriebsvorrichtung durch den Motor 4 über ein Stirnraduntersetzungsgetriebe 5a bis 5f sowie 33. Bezüglich der weiteren Details der Antriebsvorrichtung wird insbesondere auf das Ausführungsbeispiel der Figuren 1 bis 10 verwiesen.

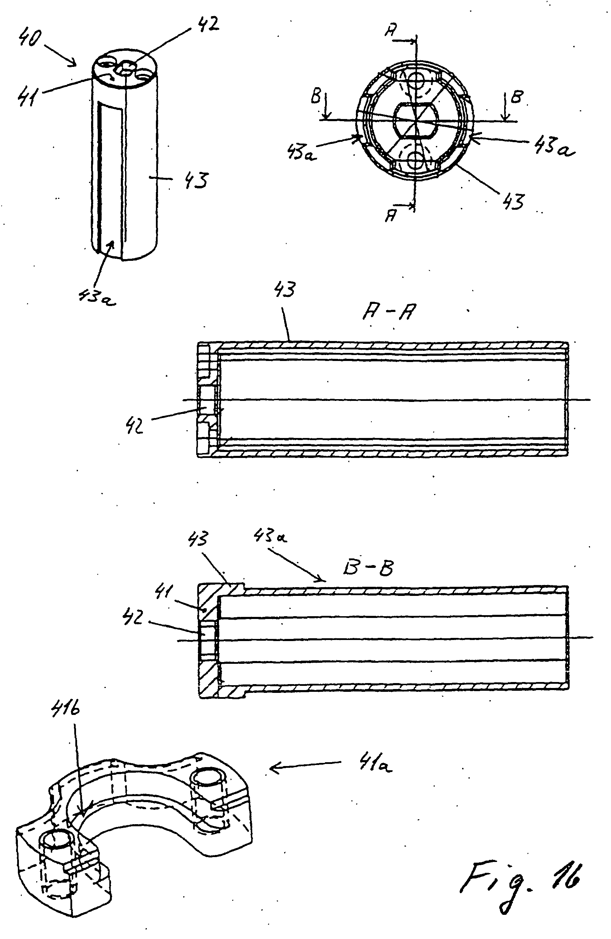

Die Figuren 15 und 16 zeigen ein drittes Ausführungsbeispiel der Erfindung mit einer dem ersten Ausführungsbeispiel im wesentlichen entsprechenden Antriebsvorrichtung. Figur 15 zeigt die Antriebsvorrichtung in einer perspektivischen Gesamtsicht, in einer Draufsicht mit zwei eingetragenen Schnitten A-A und B-B und Teile dieser beiden Schnitte. Figur 16 zeigt die Verdrehsicherung 40 in einer perspektivischen Gesamtsicht, in einer Ansicht von hinten und in den beiden in dieser Ansicht eingetragenen Schnitten A-A und B-B. Ferner ist in Figur 26 ein an der Verdrehsicherung 40 angebrachter Mitnehmer 41a dargestellt. Die Verdrehsicherung 40 ist eine Weiterentwicklung der Verdrehsicherung des ersten Ausführungsbeispiels.

Die Verdrehsicherung 40 des dritten Ausführungsbeispiels wird jedoch nicht durch eine Verdrehsicherungsgabel mit durchgreifbaren Geradführungsschlitzen gebildet. Die Verdrehsicherung 40 umgibt vielmehr den aus dem Basisteil 1 herausschiebbaren Teil der Antriebsvorrichtung, mit Ausnahme des ausgefahrenen Teils der ersten Verschiebestufe 10. Die Verdrehsicherung 40 des dritten Ausführungsbeispiels ist als geschlossener Hülsenkörper ausgebildet. Sie bietet Schutz gegen Verschmutzung. Vorzugsweise wird sie als Keramikteil gefertigt. Statt Durchgriffsschlitzen weist dieser Hülsenkörper 43 zwei in Längsrichtung des Hülsenkörpers 43 nutenförmig verlaufende Ausnehmungen 43a auf, in die jeweils Geradführungsmittel des Basisteils 1 zur Geradführung der Verdrehsicherung 40 eingreifen. Die Geradführung als solche ist der des ersten Ausführungsbeispiels vergleichbar. Im Ausführungsbeispiel sind zwei gerade, an dem hinteren Umfangsrand des Hülsenkörpers 43 auslaufende Ausnehmungen 43a vorgesehen. Grundsätzlich würde auch eine Ausnehmung genügen; es könnten jedoch auch mehr als zwei Ausnehmungen 43a vorgesehen sein.

In den Hülsenkörper 43 ist ein Mitnehmer 41 a eingesetzt und durch Verschrauben mit der einen Hülsenboden bildenden Scheibe 41 befestigt. Der Mitnehmer 41a ist, wie am besten in Figur 16 unten zu erkennen, ein teilringförmiges, im Ausführungsbeispiel halbkreisförmiges Bauteil, dessen äussere Umfangsfläche an der inneren Umfangsfläche des Hülsenkörpers 43 zwecks Positionierung der Befestigungsbohrungen teilweise eng anliegt. Der Mitnehmer 41 a bildet eine Manschette für die zweite Verschiebestufe 20 und dient zur Mitnahme der Verdrehsicherung 40 durch die zweite Verschiebestufe 20. Hierfür greift der Mitnehmer 41a mit einem radial von einer Innenumfangsfläche nach innen ragenden Flansch 41 b in eine umlaufende Ausnehmung an der äusseren Mantelfläche der zweiten Verschiebestufe 20. Die Verdrehsicherung 40 wird durch den Mitnehmer 41 a derart sowohl in Vorschubrichtung als auch gegen die Vorschubrichtung an der zweiten Verschiebestufe 20 verschiebegesichert: er behindert jedoch die Drehbewegung der zweiten Verschiebestufe 20 nicht.

## Patentansprüche

1. Vorrichtung zur Verabreichung eines Medikamentfluids, die Vorrichtung umfassend:
a) ein Gehäuse (1, G; G),
b) ein Behältnis (A) für das Medikamentfluid,
c) einen Kolben (K), durch dessen Vorschieben das Medikamentfluid dosiert aus dem Behältnis (A) verdrängt wird,
d) und eine Antriebsvorrichtung umfassend:
d1) eine Verschiebestufe (10), die dem Gehäuse (1, G; G) gegenüber verschiebbar ist und bei einem Verschieben den Kolben (K) im Behältnis (A) vorschiebt, wodurch Medikamentfluid dosiert aus dem Behältnis (A) verdrängt wird, und die ein Gewinde (15) aufweist,
d2) ein Antriebsglied (30), das von dem Gehäuse (1, G; G) drehbar gelagert und axial fixiert wird und ein Gewinde (36) aufweist,
d3) und eine Gewindehülse (20), die gegenüber dem Gehäuse (1, G; G) und auch gegenüber der Verschiebestufe (10) in Vorschubrichtung des Kolbens (K) verschiebbar ist und bei ihrem Verschieben in Vorschubrichtung des Kolbens (K) die Verschiebestufe (10) mitnimmt, und die ein Innengewinde (25) und ein Außengewinde (26) aufweist, von denen das eine (25) mit dem Gewinde (15) der Verschiebestufe (10) in einem ersten Gewindeeingriff und das andere (26) mit dem Gewinde (36) des Antriebsglieds (30) in einem zweiten Gewindeeingriff ist, wobei die Gewindehülse (20) und die Verschiebestufe (10), in Vorschubrichtung des Kolbens (K) gesehen, sich wenigstens teilweise überlappen,
**dadurch gekennzeichnet, dass**
e) das Antriebsglied (30) eine die Gewindehülse (20) umgebende Antriebshülse und sein Gewinde (36) ein mit dem Außengewinde (26) in dem zweiten Gewindeeingriff befindliches Innengewinde ist
f) und dass die Gewindehülse (20) die Verschiebestufe (10) umgibt und ihr Innengewinde (25) mit dem Gewinde (15) der Verschiebestufe (10) in dem ersten Gewindeeingriff ist.

2. Vorrichtung nach Anspruch 1, **dadurch gekennzeichnet, dass** die Antriebsvorrichtung und das Behältnis (A) je in dem gemeinsamen Gehäuse (1; G) aufgenommen und fixiert sind, dass der Kolben (K) nur vom Behältnis (A) gehalten wird und die Verschiebestufe (10) beim Verschieben gegen den Kolben (K) drückend nur an den Kolben (K) anstößt.

3. Vorrichtung nach einem der vorhergehenden Ansprüche, **dadurch gekennzeichnet, dass** die Gewindehülse (20) durch das Antriebsglied (30) sowohl mitverdrehbar als auch verschiebbar ist.

4. Vorrichtung nach dem vorhergehenden Anspruch, **dadurch gekennzeichnet, dass** das Außengewinde (26) der Gewindehülse (20) und das Gewinde (15) der Verschiebestufe (10) gleichsinnig sind.

5. Vorrichtung nach einem der vorhergehenden Ansprüche, **dadurch gekennzeichnet, dass** entweder die Verschiebestufe (10) oder die Gewindehülse (20) durch eine Verdrehsicherung (40; 40a) an einer Drehbewegung gegenüber dem Gehäuse (G; 1, G) gehindert wird.

6. Vorrichtung nach dem vorhergehenden Anspruch, **dadurch gekennzeichnet, dass** die Verdrehsicherung durch ein Gleitstück (40) gebildet wird mit wenigstens einer Gleitfläche zum Gehäuse (1, G; G) und wenigstens einer Gleitfläche zur Verschiebestufe (10), wobei diese Gleitfläche Verschiebebewegungen zulässt und ein Verdrehen der Verschiebestufe (10) gegenüber dem Gehäuse (1, G; G) verhindert.

7. Vorrichtung nach dem vorhergehenden Anspruch, **dadurch gekennzeichnet, dass** das Gleitstück (40) zusammen mit der Gewindehülse (20) gemeinsam verschiebbar ist.

8. Vorrichtung nach einem der Ansprüche 5 bis 7, **dadurch gekennzeichnet, dass** die Verdrehsicherung (40) einen Hülsenkörper (43) aufweist, der Komponenten der Antriebsvorrichtung umgibt und so vor Verschmutzung schützt.

## Claims

1. Device for dispensing a medicinal fluid, this device comprising:
a) a housing (1, G; G),
b) a container (A) for the medicinal fluid,
c) a piston (K) which, when pushed forward, causes the medicinal fluid to be displaced out of the container (A) in a dosed manner,
d) and a drive device comprising:
d1) a shifting stage (10) that can be displaced relative to the housing (1, G; G) and which, when displaced, pushes the piston (K) forward in the container (A), through which medicinal fluid is displaced out of the
container (A) in a dosed manner, and which has a screw thread (15), d2) a drive element (30) which is pivoted and axially fixed by the housing (1, G; G) and which has a screw thread (36),
d3) and a threaded bush (20) which can be displaced relative to the housing (1, G; G) and also relative to the shifting stage (10), in the forward feed direction of the piston (K), and which when pushed in the forward feed direction of the piston (K) takes the shifting stage (10) along with it, and which has an internal screw thread (25) and an external screw thread (26), of which the one (25) is in a first screw-thread connection with the screw thread (15) of the shifting stage (10) and the other (26) is in a second screw-thread connection with the screw thread (36) of the drive element (30), wherein the threaded bush (20) and the shifting stage (10), seen in the forward feed direction of the piston (K), overlap one another at least partially,
**characterised in that**
e) the drive element (30) is a drive sleeve that surrounds the threaded bush (20), and its screw thread (36) is an internal screw thread that is engaged with the external screw thread (26) in the second screw-thread connection,
f) and that the threaded bush (20) surrounds the shifting stage (10), and its internal screw thread (25) is engaged with the screw thread (15) of the shifting stage (10) in the first screw-thread connection.

2. Device according to claim 1, **characterised in that** the drive device and the container (A) are each accommodated and fixed in the common housing (1; G), that the piston (K) is held only by the container (A), and that when pushed against the piston (K), the shifting stage (10) strikes only against the piston (K).

3. Device according to one of the preceding claims, **characterised in that** the threaded bush (20) can be both rotated along with and displaced by the drive element (30).

4. Device according to the preceding claim, **characterised in that** the external screw thread (26) of the threaded bush (20) and the screw thread (15) of the shifting stage (10) go in the same direction.

5. Device according to one of the preceding claims, **characterised in that** either the shifting stage (10) or the threaded bush (20) is prevented from making a rotational movement relative to the housing (G; 1, G) by an anti-twist protection (40; 40a).

6. Device according to the preceding claim, **characterised in that** the anti-twist protection is formed by a sliding piece (40) with at least one sliding surface to the housing (1, G; G) and at least one sliding surface to the shifting stage (10), wherein this sliding surface allows displacement movements and prevents any rotation of the shifting stage (10) relative the housing (1, G; G).

7. Device according to the preceding claim, **characterised in that** the sliding piece (40) can be jointly displaced together with the threaded bush (20).

8. Device according to one of the claims 5 to 7, **characterised in that** the anti-twist protection (40) has a sleeve body (43) that surrounds components of the drive device and thus protects against contamination.

## Revendications

1. Dispositif pour l'administration d'un médicament sous forme de fluide, le dispositif comprenant :
a) un boîtier (1, G ; G),
b) un contenant (A) pour le médicament sous forme de fluide,
c) un piston (K) grâce à l'avancée duquel le médicament sous forme de fluide est chassé de manière dosée hors du contenant (A),
d) et un dispositif d'entraînement comprenant :
d1) un gradin à coulisse (10) qui peut coulisser par rapport au boîtier (1, G ; G) et qui fait avancer le piston (K) dans le contenant (A) lors d'un coulissement, ce qui chasse de manière dosée le médicament sous forme de fluide hors du contenant (A), et qui présente un filetage (15),
d2) un élément d'entraînement (30) qui est placé de façon à pouvoir être tourné par le boîtier (1, G ; G) et qui est fixé de manière axiale et qui présente un filetage (36),
d3) et une enveloppe de filetage (20) qui peut coulisser par rapport au boîtier (1, G ; G) et également par rapport au gradin à coulisse (10) suivant la direction d'avance du piston (K) et qui emporte avec elle, lorsqu'elle coulisse suivant la direction d'avance du piston (K), le gradin à coulisse (10), et qui présente un filetage interne (25) et un filetage externe (26), dont l'un (25) est dans un premier engagement avec le filetage (15) du gradin à coulisse (10) et l'autre (26) est dans un second avec le filetage (36) de l'élément d'entraînement (30), sachant que l'enveloppe de filetage (20) et le gradin à coulisse (10) se chevauchent au moins en partie, suivant la direction d'avance du piston (K),
**caractérisé en ce que**
e) l'élément d'entraînement (30) est une enveloppe d'entraînement entourant l'enveloppe de filetage (20) et son filetage (36) est un filetage interne se trouvant dans un deuxième engagement avec le filetage externe (26),
f) et **en ce que** l'enveloppe de filetage (20) entoure le gradin à coulisse (10) et son filetage interne (25) est dans le premier engagement avec le filetage (15) du gradin à coulisse (10).

2. Dispositif selon la revendication 1, **caractérisé en ce que** le dispositif d'entraînement et le contenant (A) sont logés et fixés respectivement dans le boîtier commun (1 ; G), **en ce que** le piston (K) est maintenu seulement par le contenant (A) et le gradin à coulisse (10) ne vient heurter que le piston (K) lorsqu'elle coulisse contre le piston (K) lors d'un coulissement.

3. Dispositif selon l'une quelconque des revendications précédentes, **caractérisé en ce que** l'enveloppe de filetage (20) peut être aussi bien emmenée par l'élément d'entraînement (30) en rotation que par coulissement.

4. Dispositif selon l'une quelconque des revendications précédentes, **caractérisé en ce que** le filetage externe (26) de l'enveloppe de filetage (20) et le filetage (15) du gradin à coulisse (10) présentent le même sens.

5. Dispositif selon l'une quelconque des revendications précédentes, **caractérisé en ce que** soit le gradin à coulisse (10) soit l'enveloppe de filetage (20) est bloquée en rotation par rapport au boîtier (G ; 1, G) grâce à une protection contre la rotation (40 ; 40a).

6. Dispositif selon l'une quelconque des revendications précédentes, **caractérisé en ce que** la protection contre la rotation est formée par une pièce coulissante (40) avec au moins une surface coulissante vers le boîtier (1, G ; G) et au moins une surface coulissante vers le gradin à coulisse (10) cette surface coulissante permettant des coulissements et empêche une rotation du gradin à coulisse (10) par rapport au boîtier (1, G ; G).

7. Dispositif selon l'une quelconque des revendications précédentes, **caractérisé en ce que** la pièce coulissante (40) peut être coulissée communément avec l'enveloppe de filetage (20).

8. Dispositif selon l'une quelconque des revendications 5 à 7, **caractérisé en ce que** la protection de rotation (40) présente un corps d'enveloppe (43) qui entoure des composants du dispositif d'entraînement et qui protège ainsi des salissures.
